# EUROPEAN PATENT SPECIFICATION

(11) **EP 2 797 486 B1**
(45) Date of publication and mention of the grant of the patent: **26.04.2017**
(21) Application number: 12861469.0
(22) Date of filing: 26.12.2012
(51) Int. Cl.: A61B 1/00, A61B 1/018, A61B 1/04, A61B 17/28, A61B 1/313, A61B 34/20, A61B 34/30, A61B 17/29, A61B 1/045, A61B 90/00

(54) **STEREO-ENDOSCOPE DEVICE, STEREO-ENDOSCOPE SYSTEM, AND STEREO-ENDOSCOPE ROBOT**
STEREOENDOSKOPSVORRICHTUNG, STEREOENDOSKOPSYSTEM UND STEREOENDOSKOPROBOTER
DISPOSITIF STÉRÉOENDOSCOPIQUE, SYSTÈME STÉRÉOENDOSCOPIQUE ET ROBOT STÉRÉOENDOSCOPIQUE

(30) Priority: 28.12.2011 JP 2011289876
(43) Date of publication of application: 05.11.2014
(73) Proprietor: Olympus Corporation, Tokyo 192-8507 (JP)
(72) Inventor: KIKUCHI, Satoru, Hachioji-shi, Tokyo 192-8507 (JP); KONNO, Osamu, Hachioji-shi, Tokyo 192-8507 (JP)
(74) Representative: Schicker, Silvia
(86) International application number: PCT/JP2012/084272
(87) International publication number: WO 2013/100171

(56) References cited:
- EP-A1- 2 765 899
- JP-A- H05 341 206
- JP-A- H06 105 806
- JP-A- H06 261 860
- JP-A- 2001 025 035
- JP-B2- 3 289 996
- US-A1- 2005 234 296
- US-A1- 2006 020 287
- US-A1- 2009 270 677

## Description

### {Technical Field}

The present invention relates to a stereo-endoscope device, a stereo-endoscope system, and a stereo-endoscope robot.

### {Background Art}

There are known conventional technologies for viewing an object stereoscopically with an endoscope used for surgery (see PTL 1, for example). In a technology described in PTL 1, by deforming ends of a plurality of imaging fibers that are disposed at the tip of an inserted portion inserted into the body, the convergence angle, which is the angle of intersection of the optical axes of the imaging fibers, is adjusted, thus changing the stereoscopic effect of the acquired image.

### {Citation List}

### {Patent Literature}

{PTL 1} Japanese Unexamined Patent Application, Publication No. Hei 08-94966

JP 3 289996 B2 discloses an endoscope apparatus which is suitable for observation and treatment of the inside of a body cavity of an organism and by which a part to be treated can be observed in a stereoscopic manner. This apparatus includes an endoscope which has first and second bending parts operable independently and a control means for controlling bending shapes of first and second bending parts according to the part to be observed allowing spectroscopic viewing of the part.

EP 2 765 899 A1 discloses an stereoscopic endoscope device including two image capture elements spaced apart from each other and disposed at a distal end of an insertion section to be inserted into a subject; an angle changing mechanism that changes a relative angle between optical axes of the image capture elements; a distance sensor that detects the distance from the image capture elements to the subject; and a controller that controls the angle changing mechanism on the basis of the distance detected by the distance sensor.

US 2009/270677 A1 discloses an apparatus and methods suitable for facilitating positioning one or more medical components. In one embodiment, the apparatus comprises a rail member having proximal and distal ends and a generally longitudinal axis. A first connector is adapted to be selectively coupled to the rail member, and a first medical component is adapted to be coupled to the first connector. The first connector is disposed for selective longitudinal movement along the rail member, thereby permitting movement of the first medical component with respect to the rail member. In this manner, one or more components, such as a catheter and a needle, may be inserted into a human or animal body in a controlled fashion with respect to one another.

US 2005/234296 A1 discloses methods and apparatus for obtaining endoluminal access. An elongate body is configured for insertion within a body lumen, conduit, organ, orifice or passageway, the elongate body having a working axis and a distal region, and an articulating element disposed near the distal region, the articulating element configured to articulate off-axis from the working axis of the elongate body.

### {Summary of Invention}

### {Technical Problem}

However, the technology described in PTL 1 has a disadvantage in that it is necessary for the observer, such as a doctor, to manually adjust the convergence angle by deforming the imaging fibers while viewing the monitor, and, when the observer has his/her hands full during surgery etc., it is difficult to acquire an appropriate stereoscopic image in accordance with the movement of the object.

An object of the present invention is to provide a stereo-endoscope system, and a stereo-endoscope robot capable of easily acquiring appropriate stereoscopic images of an affected site.

### {Solution to Problem}

In order to achieve the above-described object, the present invention provides a stereo-endoscope system according to claim 1 and a stere-endoscope robot according to claim 8.

According to a first aspect, a stereo-endoscope system may include: an inserted portion that can be inserted into a subject's body; two imaging devices that are disposed on the inserted portion with a space therebetween; an angle changing mechanism that changes a relative angle of optical axes of the imaging devices; a distance detecting unit for detecting the distance to the imaging devices from a treatment part of a medical device that is inserted into the subject's body and is used to treat an affected site; and a control section that controls the angle changing mechanism based on insertion information of the medical device, the insertion information including the distance detected by the distance detecting unit.

According to this aspect, the inserted portion is inserted into the subject's body, and the two imaging devices are actuated, thereby making it possible to acquire stereoscopic images of the affected site in the subject's body. In this case, when the medical device is inserted into the subject's body, the distance detecting unit detects the distance from the treatment part of the medical device to the imaging devices, and the control section controls the angle changing mechanism based on the detected distance.

Specifically, since the angle changing mechanism changes the relative angle of the optical axes of the two imaging devices, the control section adjusts the convergence angle of the two imaging devices according to the distance from the treatment part of the medical device to the imaging devices. Thus, it is possible to automatically acquire stereoscopic images with a central focus on the treatment region of the treatment part of the medical device, at the affected site in the subject's body.

In the above-described aspect, the distance detecting unit may include a measurement part that measures the amount of insertion of the treatment part of the medical device inserted into the subject's body and a conversion part that converts the amount of insertion measured by the measurement part to the distance from the treatment part of the medical device to the imaging devices.

With this structure, it is possible to easily detect the distance from the treatment part of the medical device to the imaging devices based on the amount of insertion of the treatment part of the medical device, without directly measuring this distance.

In the above-described aspect, the control section may control the angle changing mechanism such that an intersection of the optical axes of the imaging devices coincides with the position of the treatment part of the medical device.

With this structure, the intersection of the optical axes of the imaging devices can be made to coincide with the treatment part of the medical device based on the distance detected by the distance detecting unit. Thus, it is possible to automatically acquire appropriate stereoscopic images with a central focus on the treatment region of the treatment part of the medical device.

In the above-described aspect, the angle changing mechanism may include, at a tip of the inserted portion, a pair of swiveling members attached so as to be capable of swiveling on shafts that extend in a direction intersecting with the longitudinal axis of the inserted portion and driving units for swiveling the pair of swiveling members, and the imaging devices may be attached to the swiveling members.

With this structure, by actuating the driving units, constituting the angle changing mechanism, to swivel the pair of swiveling members, the relative angle of the optical axes of the two imaging devices can be easily changed.

In the above-described aspect, the driving units may swivel the pair of swiveling members between closed positions in which the pair of swiveling members extend in the direction of the longitudinal axis of the inserted portion and are close to each other and open positions in which the pair of swiveling members extend in directions intersecting with the longitudinal axis of the inserted portion and are away from each other.

With this structure, in inserting the inserted portion into the subject's body, the swiveling members are located at the closed positions to be along the longitudinal direction of the inserted portion, thereby reducing the cross-sectional area to make the insertion easier. Furthermore, after the tip of the inserted portion is inserted into the subject's body, the swiveling members are made to swivel to be located at the open positions, thus ensuring a space between the imaging devices and making it easier to form a large convergence angle.

According to a second aspect, a stereo-endoscope system may include: one of the above-described stereo-endoscope devices; the medical device that has a swiveling part capable of swiveling the treatment part on a shaft extending in a direction intersecting with the longitudinal axis of the medical device; and a swivel-angle detecting section that detects a swivel angle of the swiveling part of the medical device, in which the control section controls the angle changing mechanism based on insertion information of the medical device, the insertion information including the swivel angle detected by the swivel-angle detecting section.

According to this aspect, by swiveling the swiveling part of the medical device, the treatment region of the treatment part at the affected site in the subject's body can be changed. In this case, the control section controls the angle changing mechanism based on the swivel angle of the swiveling part of the medical device detected by the swivel-angle detecting section, thereby making it possible to automatically acquire stereoscopic images with a central focus on the treatment region of the treatment part over the entire swivel range of the swiveling part of the medical device.

In the above-described aspect, the stereo-endoscope system may further include: an inserted member that has a through-hole into which the medical device can be inserted; and an insertion-angle detecting section that detects an angle of a central axis of the through-hole of the inserted member, and the control section may control the angle changing mechanism based on insertion information of the medical device, the insertion information including the angle detected by the insertion-angle detecting section.

With this structure, by changing the angle of the central axis of the through-hole of the inserted member, the treatment region of the treatment part of the medical device at the affected site in the subject's body can be changed. In this case, the control section controls the angle changing mechanism based on the angle of the central axis of the through-hole of the inserted member detected by the insertion-angle detecting section, thereby making it possible to automatically acquire stereoscopic images of the entire treatment region of the treatment part of the medical device, which is determined by the angle of the central axis of the through-hole of the inserted member.

In the above-described aspect, the stereo-endoscope system may further include: at least one medical device identical to said medical device; and a motion detecting section that detects motions of the treatment parts of the medical devices, and the control section may control the angle changing mechanism based on the distance to the imaging devices from the treatment part of the medical device whose treatment part has the largest amount of motion detected by the motion detecting section.

Among the plurality of medical devices, the medical device whose treatment part has the largest amount of motion is considered to be the one being manipulated by the operator. Therefore, with this structure, it is possible, in an automatic manner, to preferentially acquire stereoscopic images with a central focus on the treatment region of the treatment part of the medical device that the operator focuses on, among the plurality of medical devices.

According to a second aspect, a stereo-endoscope robot may include: one of the above-described stereo-endoscope systems; a manipulation part that manipulates the medical device through remote manipulation performed by an operator; and an information detecting section that detects the insertion information of the medical device from manipulation information of the medical device manipulated by the manipulation part, in which the control section controls the angle changing mechanism based on the insertion information of the medical device detected by the information detecting section.

According to this aspect, the affected site in the subject's body can be treated via the manipulation part through remote manipulation performed by the operator. In this case, the control section controls the angle changing mechanism based on the insertion information of the medical device detected by the information detecting section from the manipulation information of the manipulation part, thereby making it possible to automatically acquire stereoscopic images with a central focus on the treatment region of the treatment part of the medical device through remote manipulation.

### {Advantageous Effects of Invention}

According to the present invention, an advantage is afforded in that appropriate stereoscopic images of the affected site can be easily acquired.

### {Brief Description of Drawings}

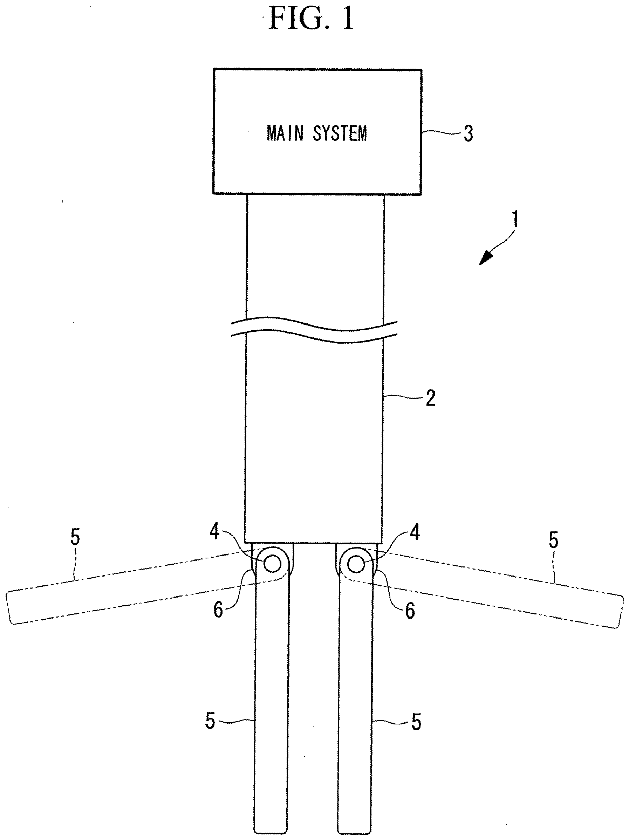

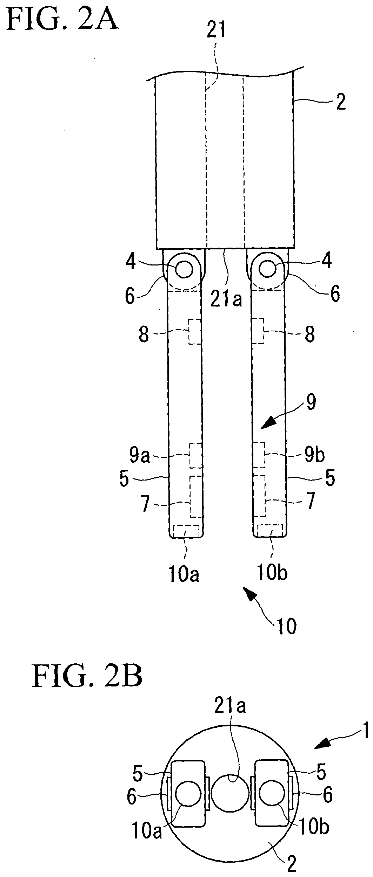

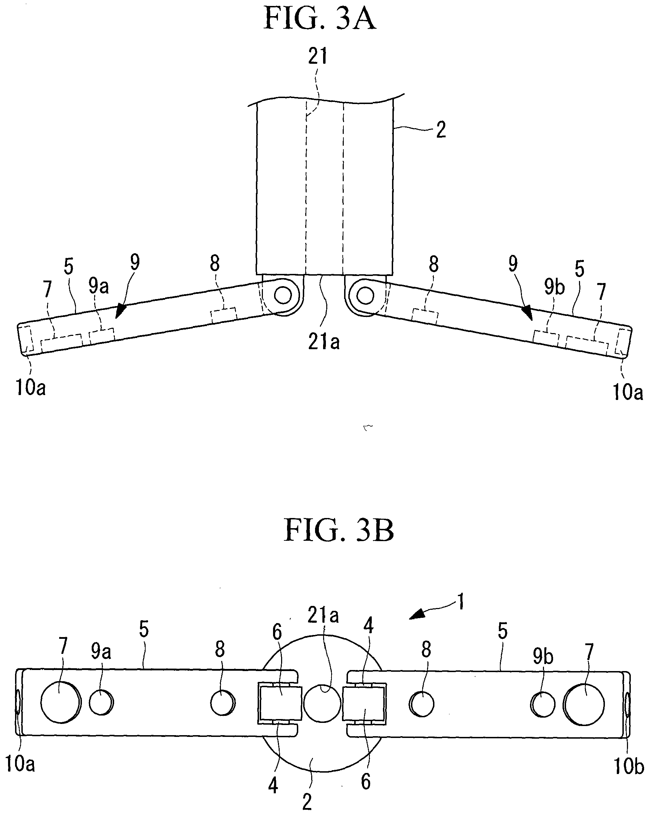

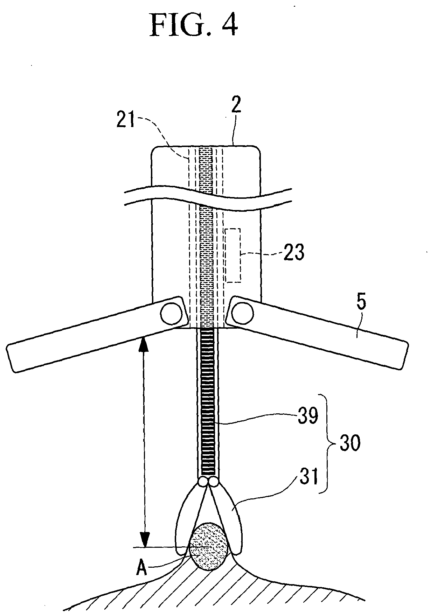

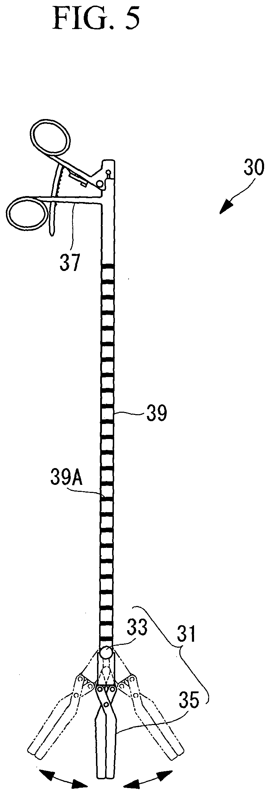

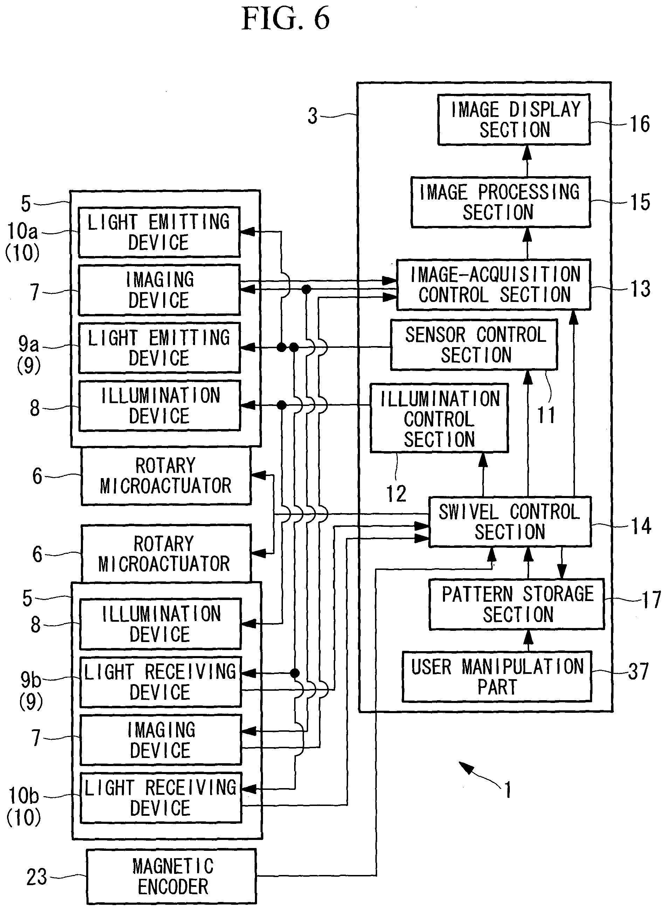

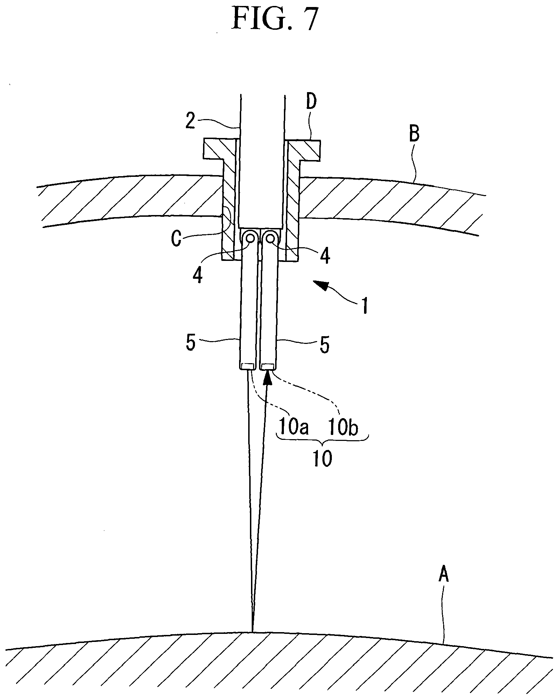

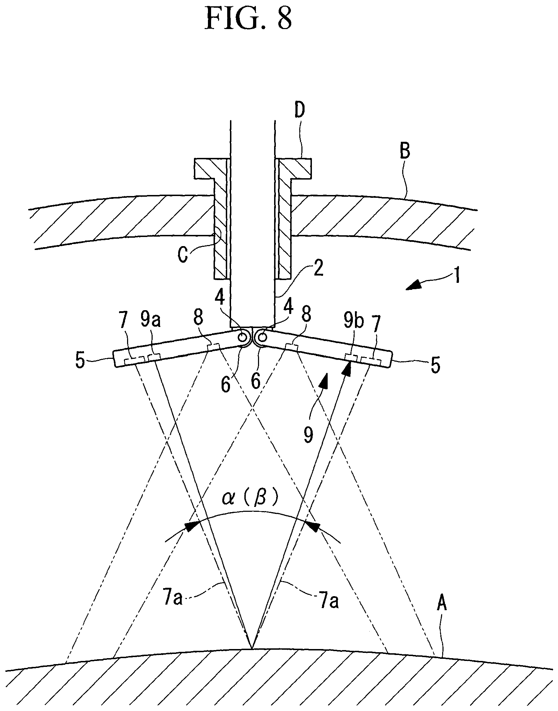

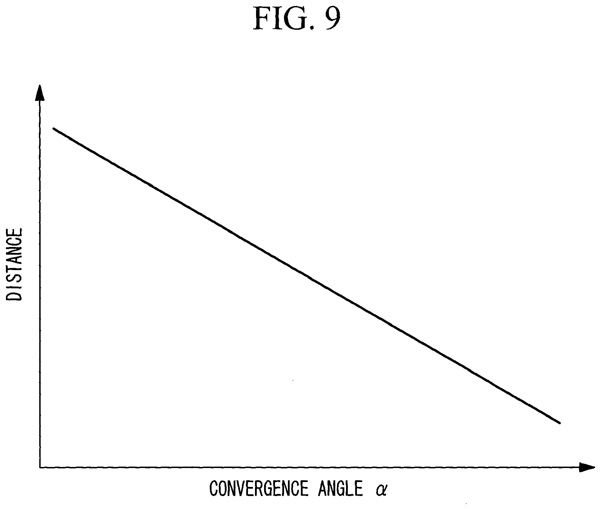

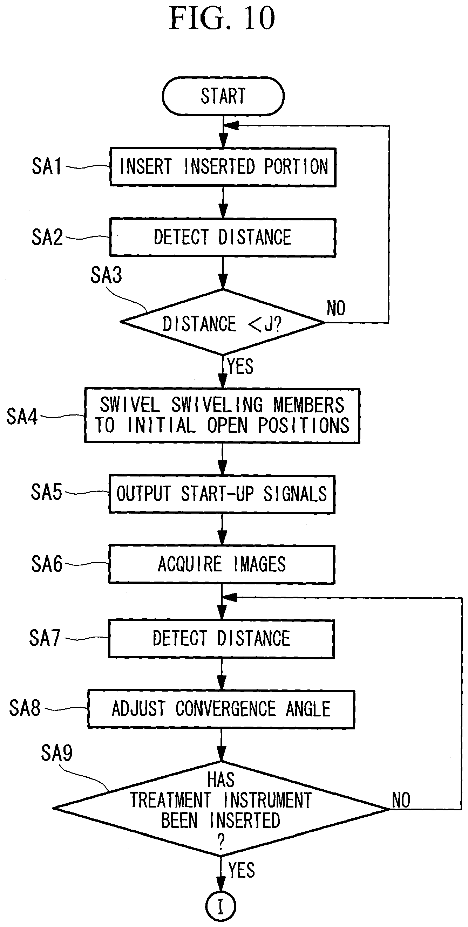

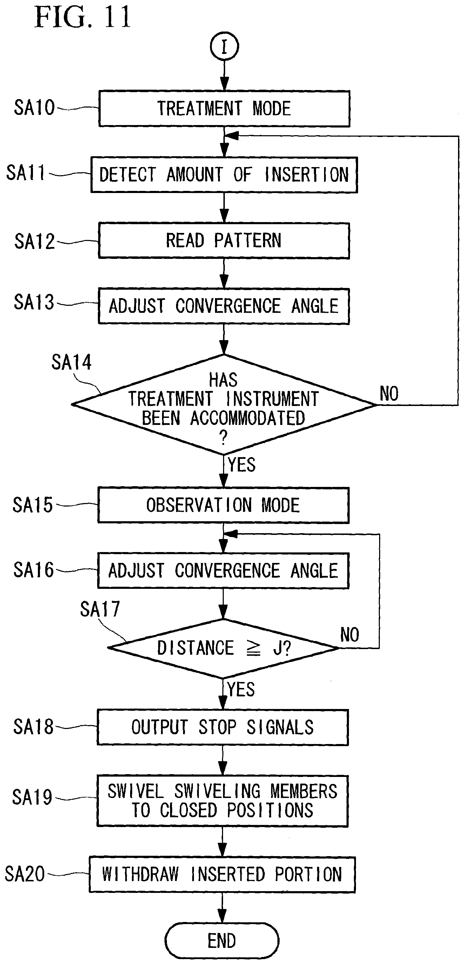

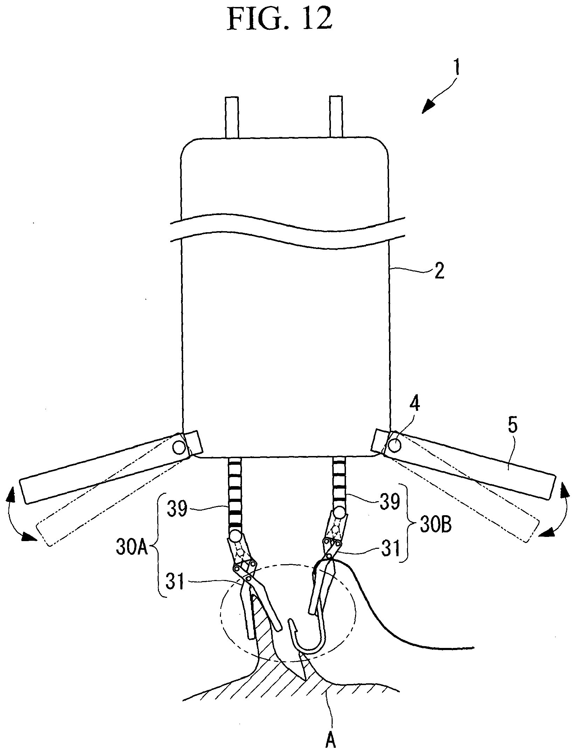

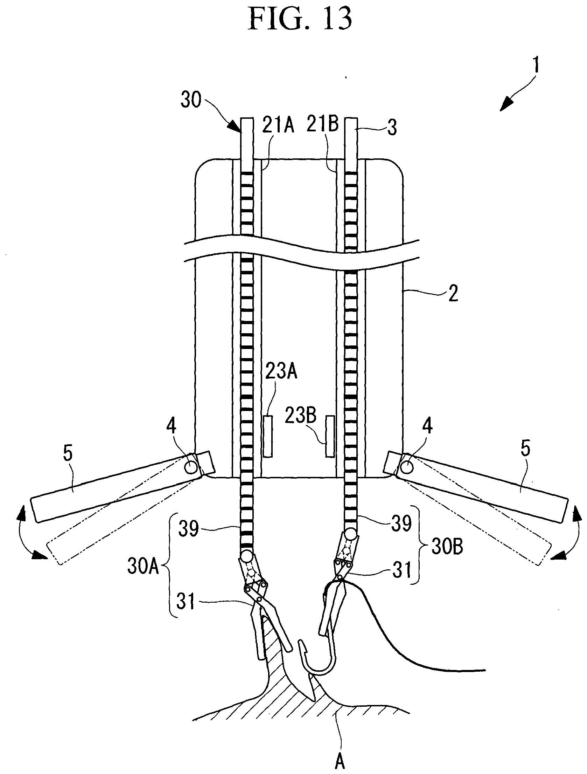

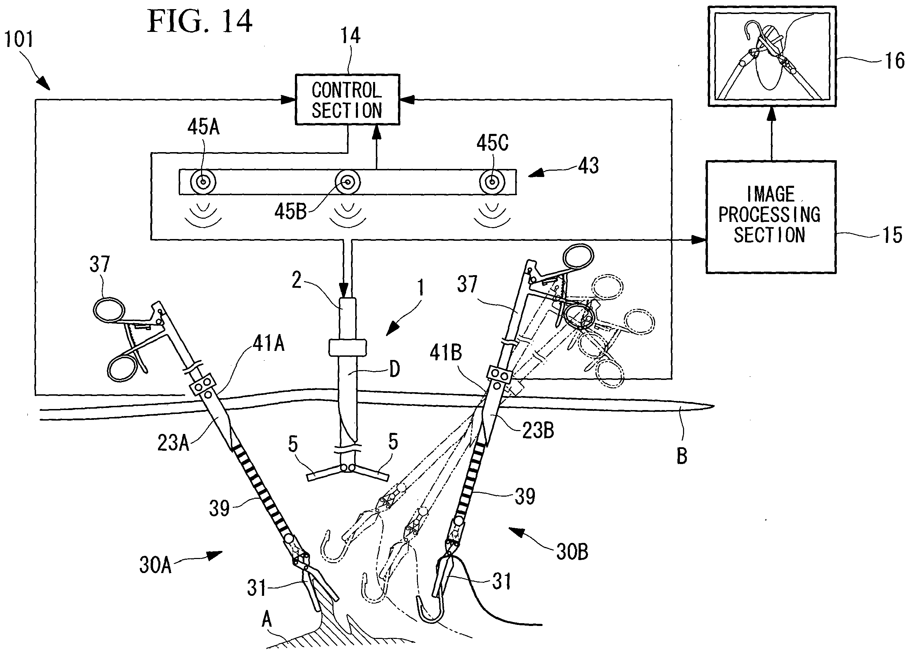

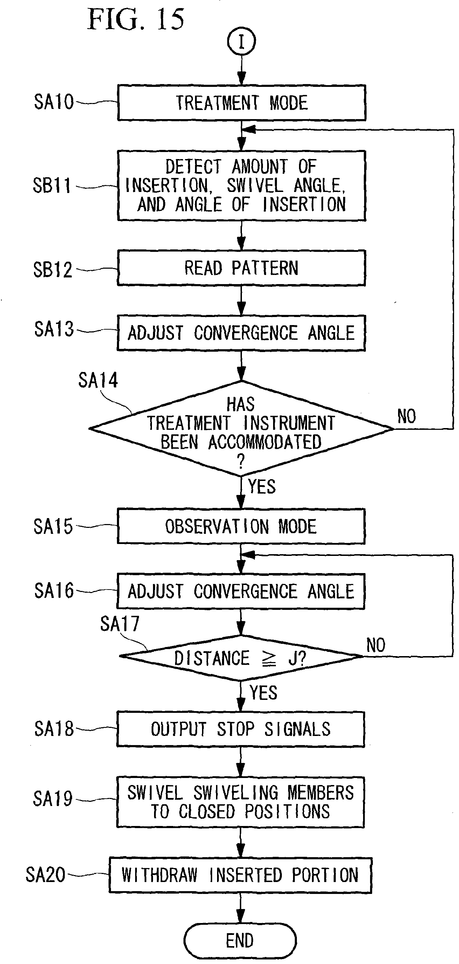

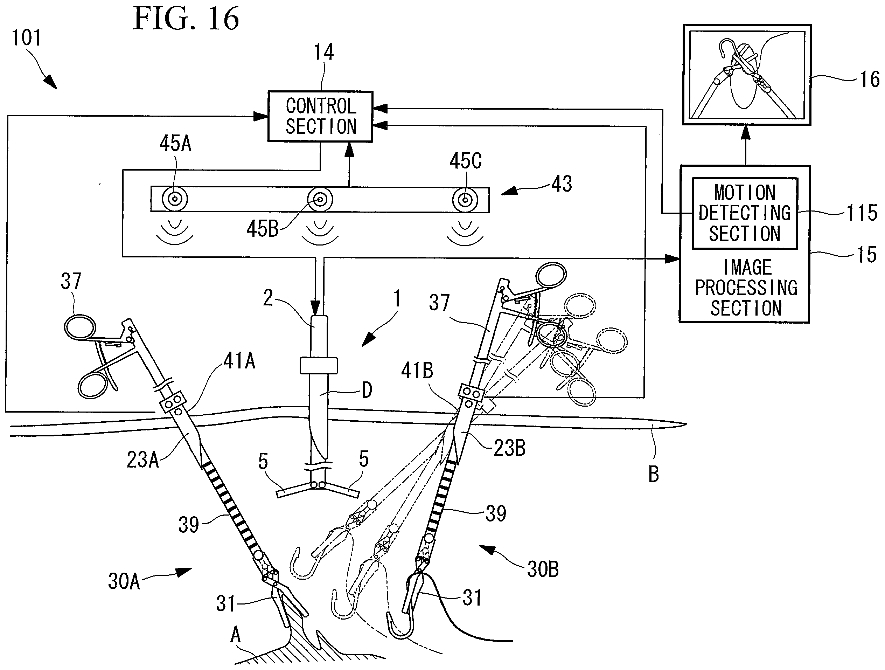

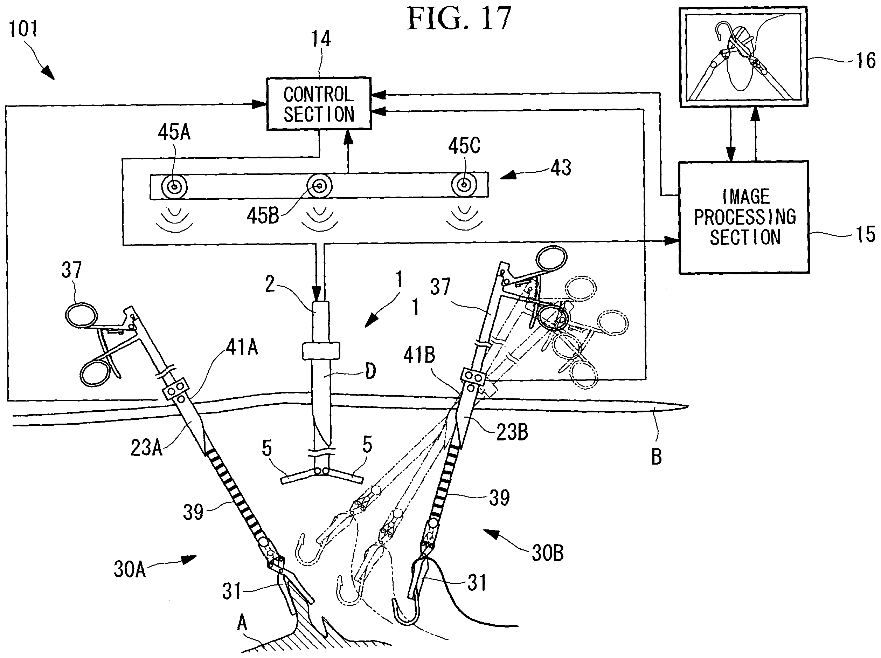

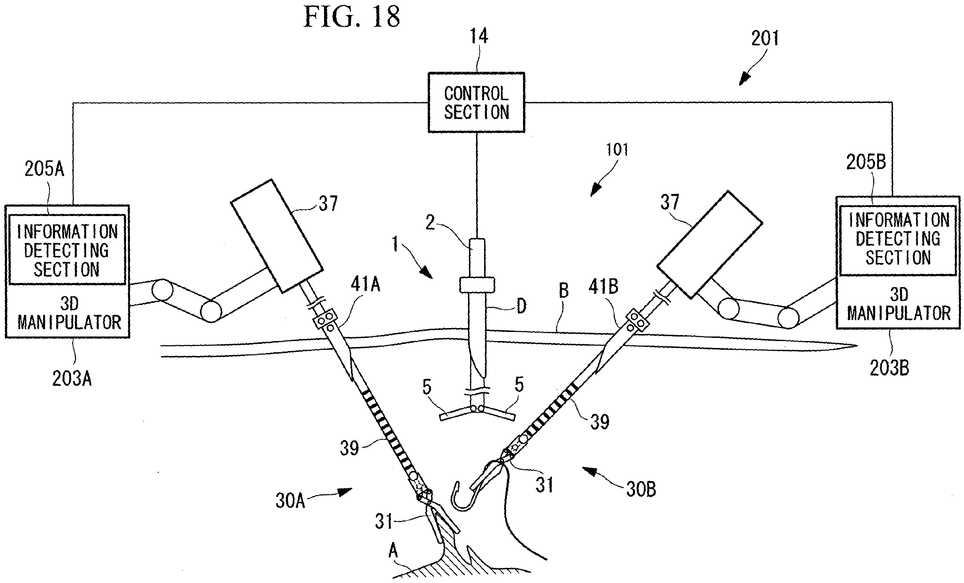

{Fig. 1} Fig. 1 is a view showing the overall structure of a stereo-endoscope device according to a first embodiment of the present invention.
{Fig. 2A} Fig. 2A is a front view of an inserted portion viewed in a radial direction when swiveling members of the stereo-endoscope device shown in Fig. 1 are located at closed positions.
{Fig. 2B} Fig. 2B is a front view of the inserted portion shown in Fig. 2A viewed from the tip thereof.
{Fig. 3A} Fig. 3A is a front view of the inserted portion viewed in a radial direction when the swiveling members of the stereo-endoscope device shown in Fig. 1 are located at open positions.
{Fig. 3B} Fig. 3B is a front view of the inserted portion shown in Fig. 3A viewed from the tip thereof.
{Fig. 4} Fig. 4 is a front view showing a state in which a treatment instrument protrudes from a channel of the inserted portion shown in Fig. 3A.
{Fig. 5} Fig. 5 is a view showing the overall structure of the treatment instrument shown in Fig. 4.
{Fig. 6} Fig. 6 is a functional block diagram of the stereo-endoscope device shown in Fig. 1.
{Fig. 7} Fig. 7 is a view showing an insertion state in which the inserted portion of the stereo-endoscope device shown in Fig. 1 is inserted into a body, and the swiveling members are located at the closed positions.
{Fig. 8} Fig. 8 is a view showing a state in which the swiveling members are located at the open positions.
{Fig. 9} Fig. 9 is a graph showing an example control pattern showing the relationship between the distance and the convergence angle, stored in a pattern storage section shown in Fig. 6.
{Fig. 10} Fig. 10 is a flowchart for explaining an observation procedure with use of the stereo-endoscope device shown in Fig. 1.
{Fig. 11} Fig. 11 is the flowchart for explaining the observation procedure continuing from Fig. 10.
{Fig. 12} Fig. 12 is a front view showing an inserted portion according to a first modification of the first embodiment of the present invention.
{Fig. 13} Fig. 13 is a longitudinal sectional view of the inserted portion shown in Fig. 12.
{Fig. 14} Fig. 14 is a view showing the overall structure of a stereo-endoscope system according to a second embodiment of the present invention.
{Fig. 15} Fig. 15 is a flowchart for explaining an observation procedure with use of the stereo-endoscope device shown in Fig. 14.
{Fig. 16} Fig. 16 is a view showing the overall structure of a stereo-endoscope system according to a third modification of the second embodiment of the present invention.
{Fig. 17} Fig. 17 is a view showing the overall structure of a stereo-endoscope system according to a fourth modification of the second embodiment of the present invention.
{Fig. 18} Fig. 18 is a view showing the overall structure of a stereo-endoscope robot according to a third embodiment of the present invention.

### {Description of Embodiments}

### First Embodiment

A stereo-endoscope device according to a first embodiment of the present invention will be described below with reference to the drawings.

As shown in Fig. 1, a stereo-endoscope device 1 of this embodiment includes an elongated inserted portion 2 that can be inserted into the body of a patient (subject's body) and a main system 3 that is connected to the inserted portion 2.

The inserted portion 2 has, on the tip thereof, two shafts (rotational axes) 4 that extend parallel to each other in a direction perpendicular to a longitudinal axis of the inserted portion 2 and two swiveling members (angle changing mechanism) 5 that are supported so as to be capable of swiveling on these shafts 4.

Rotary microactuators (driving units, angle changing mechanism) 6 that rotate the shafts 4 are provided at base ends of the swiveling members 5. When the two rotary microactuators 6 are synchronized to rotate the shafts 4 in opposite directions, the swiveling members 5 are made to swivel between closed positions in which they are located close to each other in the direction of the longitudinal axis of the inserted portion 2, as shown in Figs. 2A and 2B, and open positions in which they are located away from each other in directions intersecting with the longitudinal axis of the inserted portion 2, as shown in Figs. 3A and 3B.

The swiveling members 5 include distance sensors (distance detecting units) 9, illumination devices 8, and imaging devices 7 that face each other when the swiveling members 5 are located at the closed positions.

The imaging devices 7 are, for example, CCDs or CMOS imagers having optical axes 7a (see Fig. 8) that are perpendicular to longitudinal axes of the swiveling members 5.

The illumination devices 8 are, for example, LEDs that emit illumination light in directions intersecting with the longitudinal axes of the swiveling members 5.

The distance sensor 9 includes, for example, a light emitting device 9a that is provided on one of the swiveling members 5 and a light receiving device 9b that is provided on the other one of the swiveling members 5.

Light emitted from the light emitting device 9a is reflected on an object A (see Fig. 4 etc.), such as an affected site, of a patient and is received by the light receiving device 9b. The amount of light received by the light receiving device 9b is small when the distance is long and is large when the distance is short; therefore, the distance from the distance sensor 9 to the object A can be detected based on the amount of received light.

Furthermore, the swiveling members 5 have, at the tips thereof, a tip distance sensor (distance detecting unit) 10 that detects the distance from the object A to the swiveling members 5 in the state where the swiveling members 5 are located at the closed positions. Like the distance sensor 9, the tip distance sensor 10 includes a light emitting device 10a and a light receiving device 10b.

Furthermore, the inserted portion 2 is provided with a channel 21 that passes through the inserted portion 2 along the longitudinal axis thereof and a magnetic encoder 23 (see Fig. 4) that is disposed near the channel 21.

The channel 21 has an opening 21a between the two shafts 4, which are provided at the tip of the inserted portion 2. As shown in Fig. 4, the inserted portion 2 is structured such that a treatment instrument (medical device) 30 can be inserted into the channel 21 and made to move out of and into the opening 21a.

As shown in Fig. 5, the treatment instrument 30 includes, for example, a treatment part 31 that is used to treat the affected site of the patient, a manipulation part 37 that is grasped by an operator to manipulate the treatment part 31, and a shaft part 39 that connects the treatment part 31 and the manipulation part 37.

The treatment part 31 includes a swiveling part 33 that is provided at the tip of the shaft part 39 so as to be capable of swiveling on a swivel shaft that extends in a direction intersecting with the long axis of the shaft part 39 and a pair of forceps members 35 that extend from the swiveling part 33 toward the distal end and that can be opened and closed with an opening-and-closing shaft that extends parallel to the above-described swivel shaft of the swiveling part 33.

The operator can manipulate the manipulation part 37 to swivel the swiveling part 33 and to open and close the pair of forceps members 35.

The shaft part 39 is provided with a plurality of magnetic codes 39A that are formed of a magnetic material and arranged at regular intervals in the longitudinal direction thereof.

The magnetic encoder 23 produces a magnetic field and reads the magnetism that changes when the magnetic codes 39A on the treatment instrument 30 pass through the magnetic field, thereby detecting the amount of insertion of the treatment instrument 30. The detected amount of insertion of the treatment instrument 30 is sent to the main system 3 as insertion information. The amount of insertion of the treatment instrument 30 corresponds to the amount of protrusion of the treatment part 31 of the treatment instrument 30 from the opening 21a of the channel 21 in the inserted portion 2, for example.

As shown in Fig. 6, the main system 3 includes a sensor control section 11 that controls the distance sensor 9 and the tip distance sensor 10, an illumination control section 12 that controls lighting of the illumination devices 8, an image-acquisition control section 13 that controls image acquisition performed by the imaging devices 7, a swivel control section (control section) 14 that controls the angles of the swiveling members 5, an image processing section 15 that processes image signals acquired by the imaging devices 7, an image display section 16 that displays images processed by the image processing section 15, a pattern storage section 17 that stores a control pattern associating the distance and the convergence angle in the form of a LUT (look-up table), and the manipulation part 37 manipulated by the operator.

The swivel control section 14 switches between an observation mode and a treatment mode based on the insertion information of the treatment instrument 30 sent from the magnetic encoder 23. Specifically, the swivel control section 14 sets the observation mode when it receives insertion information indicating a state in which the amount of insertion of the treatment instrument 30 is zero, that is, a state in which the treatment part 31 of the treatment instrument 30 is accommodated in the channel 21 of the inserted portion 2. Furthermore, the swivel control section 14 sets the treatment mode when it receives insertion information indicating a state in which the amount of insertion of the treatment instrument 30 is not zero, that is, a state in which the treatment part 31 of the treatment instrument 30 protrudes from the channel 21 of the inserted portion 2. For example, in the observation mode, the inserted portion 2 is inserted into the body of the patient and is located at a desired position, and, in the treatment mode, the treatment instrument 30 is used to treat the affected site of the patient.

In the observation mode, the swivel control section 14 actuates the tip distance sensor 10 at the time of insertion during which the swiveling members 5 are located at the closed positions, as shown in Fig. 7, and actuates the distance sensor 9 when the swiveling members 5 are located at the open positions, as shown in Fig. 8.

The light receiving devices 9b and 10b, which constitute the distance sensor 9 and the tip distance sensor 10, respectively, each output a voltage signal corresponding to the amount of received light, to the swivel control section 14. The swivel control section 14 monitors the voltage signal sent from the light receiving device 10b, constituting the tip distance sensor 10, at the time of insertion during which the swiveling members 5 are located at the closed positions. Then, when the voltage signal exceeds a stored predetermined threshold, that is, when the distance between the tips of the swiveling members 5 and the object A reaches a predetermined distance, the swivel control section 14 actuates the rotary microactuators 6 to make the swiveling members 5 swivel from the closed positions to initial open positions.

Furthermore, after the swiveling members 5 are made to swivel to the initial open positions, the swivel control section 14 outputs start-up signals to the sensor control section 11, the illumination control section 12, and the image-acquisition control section 13.

Upon reception of the start-up signal, the sensor control section 11 drives the distance sensor 9, instead of the tip distance sensor 10, to make the distance sensor 9 detect the distance to the object A.

Upon reception of the start-up signal, the illumination control section 12 actuates the illumination devices 8. Upon reception of the start-up signal, the image-acquisition control section 13 actuates the imaging devices 7.

Furthermore, when the voltage signal is received from the light receiving device 9b constituting the distance sensor 9, the swivel control section 14 calculates the distance corresponding to the received voltage signal and searches for a convergence angle α corresponding to this distance, in the control pattern stored in the pattern storage section 17. Then, the swivel control section 14 drives the rotary microactuators 6 so as to achieve the found convergence angle α.

The control pattern stored in the pattern storage section 17 has a linear relationship between the distance and the convergence angle α, as shown in Fig. 9, for example. In this relationship, at a position coincident with the surface of the object A, which is located respective distances away from the imaging devices 7, the optical axes 7a of the two imaging devices 7 intersect at a corresponding convergence angle.

Furthermore, the pattern storage section 17 stores the relationship between a convergence angle P and the amount of insertion of the treatment instrument 30, which is converted to the distance from the treatment part 31 to the imaging devices 7 and then associated with the convergence angle.

By using the convergence angle α found from the control pattern based on the distance detected by the distance sensor 9, the swivel control section 14 calculates the angles of the swiveling members 5 for achieving the found convergence angle α and performs control such that the shafts 4 of the rotary microactuators 6 are rotated to these angles from the initial open positions.

On the other hand, in the treatment mode, when the insertion information of the treatment instrument 30 is received from the magnetic encoder 23, the swivel control section 14 searches, in the control pattern stored in the pattern storage section 17, for the convergence angle β corresponding to the amount of insertion of the treatment instrument 30 and drives the rotary microactuators 6 so as to achieve the found convergence angle β.

The behavior of the thus-structured stereo-endoscope device 1 of this embodiment will be described below with reference to a flowchart shown in Figs. 10 and 11.

To observe the object A in the body by using the stereo-endoscope device 1 of this embodiment, first, the rotary microactuators 6 are actuated to locate the two swiveling members 5 at the closed positions shown in Figs. 2A and 2B. In this state, as shown in Fig. 7, the inserted portion 2 is inserted, starting from the tips of the swiveling members 5, into the space in the body via a through-hole of a trocar D located in an opening C that is formed to penetrate a body surface tissue B (Step SA1). At this time, the swivel control section 14 has set the observation mode.

Next, the sensor control section 11 actuates the light emitting device 10a and the light receiving device 10b of the tip distance sensor 10. Then, a voltage signal indicating the amount of light emitted from the light emitting device 10a, reflected on the object A in the body, and then received by the light receiving device 10b is input to the swivel control section 14, and the swivel control section 14 determines the distance (Step SA2).

If the distance detected by the tip distance sensor 10 becomes smaller than a predetermined distance J ("Yes" in Step SA3), at this position, the swivel control section 14 actuates the rotary microactuators 6 to make the two swiveling members 5 swivel to the predetermined initial open positions, as shown in Fig. 8 (Step SA4).

Furthermore, at the same time that the swivel control section 14 makes the swiveling members 5 swivel to the initial open positions, the swivel control section 14 outputs start-up signals to the sensor control section 11, the illumination control section 12, and the image-acquisition control section 13 (Step SA5). Upon reception of the start-up signal, the sensor control section 11 stops the tip distance sensor 10 that has been activated and actuates the light emitting device 9a and the light receiving device 9b of the distance sensor 9.

Upon reception of the start-up signal, the illumination control section 12 actuates the illumination devices 8 to radiate illumination light onto the object A. Upon reception of the start-up signal, the image-acquisition control section 13 actuates the two imaging devices 7 to acquire image signals. The image signals acquired by the two imaging devices 7 are displayed on the image display section 16 via the image processing section 15. Thus, two types of images of the object A acquired from two directions, namely, the right and left directions, are obtained (Step SA6).

The voltage signal corresponding to the amount of light received by the light receiving device 9b of the distance sensor 9 is sent to the swivel control section 14, and the distance is calculated therein (Step SA7). The swivel control section 14 uses the calculated distance to search, in the control pattern stored in the pattern storage section 17, for the corresponding convergence angle α and calculates the angles of the swiveling members 5 for achieving the found convergence angle α. Then, the swivel control section 14 actuates the rotary microactuators 6 until the swiveling members 5 swivel so as to achieve the calculated angles (Step SA8).

The control pattern is stored so as to associate the convergence angle α and the distance at which the intersection of the optical axes 7a of the two imaging devices 7 coincides with the surface of the object A; therefore, by swiveling the swiveling members 5 so as to achieve the convergence angle α corresponding to the detected distance, it is possible to acquire two images of the object A viewed from two directions, namely, the right and left directions, with the intersection of the optical axes 7a of the two imaging devices 7 always coinciding with the surface of the object A.

Then, as shown in Fig. 4, when the treatment instrument 30 is made to protrude from the channel 21 of the inserted portion 2 and inserted into the body of the patient (Step SA9), the magnetic encoder 23 inputs, to the swivel control section 14, insertion information indicating that the amount of insertion of the treatment instrument 30 is not zero, and the swivel control section 14 switches to the treatment mode (Step SA10). Then, when the treatment instrument 30 is located near the affected site of the patient, the magnetic encoder 23 detects the amount of insertion of the treatment instrument 30 (Step SA11) and inputs the insertion information of the treatment instrument 30 to the swivel control section 14.

The swivel control section 14 searches, in the control pattern stored in the pattern storage section 17, for the relationship value (pattern) of the convergence angle P corresponding to the amount of insertion of the treatment instrument 30 (Step SA12) and drives the rotary microactuators 6 so as to achieve the found convergence angle P (Step SA13).

The control pattern is stored so as to associate the convergence angle β and the distance at which the intersection of the optical axes 7a of the two imaging devices 7 coincides with the treatment part 31 of the treatment instrument 30; therefore, by swiveling the swiveling members 5 so as to achieve the convergence angle P corresponding to the detected distance, it is possible to acquire two images of the object A viewed from two directions, namely, the right and left directions, with the intersection of the optical axes 7a of the two imaging devices 7 always coinciding with the treatment part 31 of the treatment instrument 30.

Until the amount of insertion of the treatment instrument 30, detected by the magnetic encoder 23, becomes zero, that is, until the treatment part 31 of the treatment instrument 30 is accommodated in the channel 21 of the inserted portion 2 (Step SA14), detection of the amount of insertion, reading of the control pattern, and adjustment of the convergence angle β, which are performed in Steps SA11 to SA13, are repeated. Thereafter, once the treatment instrument 30 is accommodated in the channel 21 ("Yes" in Step SA14), the swivel control section 14 switches to the observation mode (Step SA15).

Based on the relationship value for control of the swivel angles of the swiveling members 5 with respect to the amount of change in the distance, which is stored in advance, the swivel control section 14 drives the rotary microactuators 6 according to the amount of change in the distance from the treatment part 31 of the treatment instrument 30 to the imaging devices 7 (Step SA16). Then, if the inserted portion 2 is withdrawn, so that the distance to the object A is equal to or larger than the predetermined distance J ("Yes" in Step SA17), the swivel control section 14 outputs signals indicating the end of image acquisition, to the illumination control section 12 and the image-acquisition control section 13 (Step SA18). Thus, the illumination devices 8 and the imaging devices 7 are stopped, thus ending image acquisition.

Next, the swivel control section 14 actuates the rotary microactuators 6 to locate the two swiveling members 5 at the closed positions (Step SA19) and instructs the sensor control section 11 to stop the distance sensor 9 and actuate the tip distance sensor 10. Thus, the inserted portion 2 and the swiveling members 5 can be withdrawn from the body via the trocar D (Step SA20).

In this way, according to the stereo-endoscope device 1 of this embodiment, when the treatment instrument 30 is inserted into the subject's body, the swiveling members 5 are made to swivel based on the distance from the treatment part 31 to the imaging devices 7 corresponding to the amount of insertion of the treatment instrument 30 detected by the magnetic encoder 23, thereby adjusting the convergence angle β such that the intersection of the optical axes 7a of the two imaging devices 7 always coincides with the treatment part 31. Thus, it is possible to automatically acquire stereoscopic images with a central focus on the treatment region of the treatment part 31 of the treatment instrument 30 at the affected site in the subject's body, without the observer, such as a doctor, having to manually adjust the convergence angle P while viewing the image display section 16.

This embodiment can be modified as described below.

For example, in this embodiment, a description has been given of an example structure in which a single treatment instrument 30 is provided; however, in a first modification, two treatment instruments 30 may be used, as shown in Figs. 12 and 13.

In this case, the inserted portion 2 includes channels 21A and 21B that are parallel to each other and magnetic encoders 23A and 23B that are disposed near the channels 21A and 21B, respectively, and the magnetic encoders 23A and 23B detect the amounts of insertion of the treatment instruments 30A and 30B into the channels 21A and 21B, respectively.

Furthermore, the amounts of motion of the treatment parts 31 may be detected from image processing and the swivel angles of the swiveling parts 33, and the swivel angles of the swiveling members 5 may be adjusted based on the distance to the imaging devices 7 from the treatment part 31 of the treatment instrument 30A or 30B having a larger amount of motion. Alternatively, the operator may manually select one of the treatment instruments 30A and 30B and adjust the swivel angles of the swiveling members 5 based on the insertion information sent from the corresponding magnetic encoder 23A or 23B that detects the amount of insertion of the selected treatment instrument 30A or 30B.

It is assumed that the channels 21A and 21B correspond to the channel 21, the magnetic encoders 23A and 23B correspond to the magnetic encoder 23, and the treatment instruments 30A and 30B correspond to the treatment instrument 30.

When the plurality of treatment instruments 30A and 30B are used, the treatment instrument 30A or the treatment instrument 30B whose treatment part 31 has the largest amount of motion is considered to be the one being manipulated by the operator. According to this modification, it is possible, in an automatic manner, to preferentially acquire stereoscopic images with a central focus on the treatment region of the treatment part 31 of the treatment instrument 30A or the treatment instrument 30B that the operator focuses on, among the plurality of treatment instruments 30A and 30B.

In this embodiment and the first modification, which does not form part of the subject-matter of the invention, a description has been given of an example case where only the amount of insertion of the treatment instrument 30 is used as insertion information; however, in a second modification, which does form part of the subject-matter of the invention, the amount of insertion of the treatment instrument 30 and the swivel angle of the swiveling part 33 may be used as insertion information. In this case, a swivel-angle detecting section (not shown) that detects the swivel angle of the swiveling part 33 is provided on the treatment instrument 30, and the detected swivel angle is input to the swivel control section 14.

By swiveling the swiveling part 33 of the treatment instrument 30, the treatment region of the treatment part 31 at the affected site of the patient can be changed without changing the position of the inserted portion 2. According to this modification, the swivel control section 14 adjusts the swivel angles of the swiveling members 5 based on the swivel angle of the swiveling part 33 in addition to the amount of insertion of the treatment instrument 30, thereby making it possible to automatically acquire stereoscopic images with a central focus on the treatment region of the treatment part 31 over the entire swivel range of the swiveling part 33 of the treatment instrument 30.

### Second Embodiment

Next, a stereo-endoscope system according to a second embodiment of the present invention will be described.

As shown in Fig. 14, a stereo-endoscope system 101 of this embodiment includes the stereo-endoscope device 1, the two treatment instruments (medical devices) 30A and 30B, trocars (inserted members) 41A and 41B for inserting the treatment instruments 30A and 30B into the body of the patient, and an insertion-angle detecting section 43 that detects the angles of insertion of the treatment instruments 30A and 30B.

Hereinafter, identical symbols are assigned to parts having the same structures as those in the stereo-endoscope device 1 according to the first embodiment, and a description thereof will be omitted.

The trocars 41A and 41B are substantially-tubular members having through-holes into which the treatment instruments 30A and 30B can be inserted. By inserting the trocars 41A and 41B into holes incised in the surface of the patient's body, the treatment instruments can be inserted into the body of the patient. With the trocars 41A and 41B inserted into the surface of the patient's body, the angles of insertion of the trocars 41A and 41B into the body surface can be changed by using the body surface as supporting points. Furthermore, the trocars 41A and 41B are respectively provided with magnetic encoders 23A and 23B that are located near the through-holes.

The treatment instruments 30A and 30B are inserted into the body of the patient via the trocars 41A and 41B, respectively. The treatment instruments 30A and 30B are provided with swivel-angle detecting sections (not shown) for detecting the swivel angles of the swiveling parts 33. The swivel angles detected by the swivel-angle detecting sections are input to the swivel control section 14.

The insertion-angle detecting section 43 includes three cameras 45A, 45B, and 45C that acquire images of the trocars 41A and 41B inserted into the surface of the patient's body and an analysis section (not shown) that analyzes the images acquired by the cameras 45A, 45B, and 45C to detect the angles of the central axes of the through-holes of the trocars 41A and 41B (hereinafter, simply referred to as "the angles of the trocars 41A and 41B"). The angles of the trocars 41A and 41B detected by the analysis section are input to the swivel control section 14 as insertion information.

The swivel control section 14 controls the rotary microactuators 6 based on the insertion information of the treatment instruments 30A and 30B, the insertion information including the amounts of insertion of the treatment instruments 30A and 30B, which are sent from the magnetic encoders 23A and 23B, the swivel angles of the swiveling parts 33, which are sent from the swivel-angle detecting sections, and the angles of the trocars 41A and 41B, which are sent from the analysis section of the insertion-angle detecting section 43.

Specifically, the pattern storage section 17 stores the relationship between the convergence angle β and the insertion information of each of the treatment instruments 30A and 30B, which is obtained after the amount of insertion of the corresponding treatment instrument 30A and 30B, the swivel angle of the corresponding swiveling part 33, and the angle of the corresponding one of the trocars 41A and 41B are converted to the distance from the corresponding treatment part 31 to the imaging devices 7 and then associated with the convergence angle. Based on the insertion information of one of the treatment instruments 30A and 30B specified by the user, the swivel control section 14 drives the rotary microactuators 6 so as to achieve the convergence angle P corresponding to that insertion information included in control pattern.

The behavior of the thus-structured stereo-endoscope system 101 of this embodiment will be described below with reference to a flowchart shown in Fig. 15.

To perform suturing while observing the affected site of the patient by using the stereo-endoscope system 101 of this embodiment, the stereo-endoscope device 1 is inserted into the body of the patient, and the trocars 41A and 41B are inserted into the body surface B of the patient. Since the observation mode is the same as that in the first embodiment, a description thereof will be omitted.

After the inserted portion 2 of the stereo-endoscope device 1 is located at a desired position in the body of the patient, when the treatment instruments 30A and 30B are inserted into the body of the patient via the trocars 41A and 41B, respectively, the magnetic encoders 23A and 23B output the insertion information of the treatment instruments 30A and 30B, respectively, and the swivel control section 14 switches to the treatment mode (Step SA10).

In this embodiment, for example, the affected site of the patient is grasped with the treatment instrument 30A, and a needle is held with the treatment part 31 of the treatment instrument 30B. Furthermore, the rotary microactuators 6 are controlled based on the insertion information of the treatment part 31 of one of the treatment instrument 30A and the treatment instrument 30B selected by the operator.

In the treatment mode, the magnetic encoders 23A and 23B detect the amounts of insertion of the treatment instruments 30A and 30B, the swivel-angle detecting sections detect the swivel angles of the swiveling parts 33, and the insertion-angle detecting section 43 detects the angles of insertion of the trocars 41A and 41B (Step SB11). Then, the insertion information of each of the treatment instruments 30A and 30B is input to the swivel control section 14.

The swivel control section 14 searches, in the control pattern stored in the pattern storage section 17, for a relationship value (pattern) of the convergence angle P corresponding to the amount of insertion of the treatment instrument 30A or 30B, the swivel angle of the corresponding swiveling part 33, and the angle of insertion of the corresponding one of the trocars 41A and 41B (Step SB12) and drives the rotary microactuators 6 so as to achieve the found convergence angle P (Step SA13).

The control pattern is stored so as to associate the convergence angle P, the angle of insertion, the swivel angle, and the distance at which the intersection of the optical axes 7a of the two imaging devices 7 coincides with the treatment part 31 of the treatment instrument 30A or 30B; therefore, by swiveling the swiveling members 5 so as to achieve the convergence angle β corresponding to the detected distance, swivel angle, and angle of insertion, it is possible to acquire two images of the object A viewed from two directions, namely, the right and left directions, with the intersection of the optical axes 7a of the two imaging devices 7 always coinciding with the treatment part 31 of the treatment instrument 30A or 30B.

Step SA14 to Step SA20 are the same as those in the first embodiment.

According to the stereo-endoscope system 101 of this embodiment, by swiveling the swiveling part 33 of the treatment instrument 30A or 30B or by changing the angle of the trocar 41A or 41B, it is possible to change the treatment region of the treatment part 31 of the treatment instrument 30A or 30B at the affected site of the patient. The swivel control section 14 controls the angles of the swiveling members 5 based on the distance from the treatment part 31 to the imaging devices 7, the swivel angle of the swiveling part 33, and the angle of the trocar 41A or 41B, thereby making it possible to automatically acquire stereoscopic images over the entire treatment region of the treatment part 31 of the treatment instrument 30A or 30B, which is determined by the entire swivel range of the swiveling part 33 and the angle of the trocar 41A or 41B.

This embodiment can be modified as described below.

In this embodiment, the swivel control section 14 controls the rotary microactuators 6 based on the insertion information of the treatment instrument 30A or the treatment instrument 30B that is specified in advance by the operator, among the two treatment instruments 30A and 30B; however, in a third modification, whether to use the insertion information of the treatment instrument 30A or 30B may be automatically determined by the swivel control section 14, for example.

Specifically, as shown in Fig. 16, the image processing section 15 may include a motion detecting section 115 that detects, in the image, the motions of the treatment parts 31 of the treatment instruments 30A and 30B and may input motion information indicating the detected amounts of motion of the treatment parts 31 to the swivel control section 14. In this case, the swivel control section 14 selects, from the received motion information, the treatment instrument 30A or the treatment instrument 30B that has a larger amount of motion and drives the rotary microactuators 6 so as to achieve the convergence angle β corresponding to the insertion information of the selected treatment instrument 30A or treatment instrument 30B, included in the control pattern.

By doing so, it is possible to acquire, while automatically giving priority to and automatically switching to, stereoscopic images with a central focus on the treatment region of the treatment part 31 of the treatment instrument 30A or the treatment instrument 30B that the operator focuses on, among the two treatment instruments 30A and 30B.

In this modification, an information storage section (not shown) that temporarily stores the motion information output from the motion detecting section 115 may be provided. In this case, the swivel control section 14 reads the motion information stored in the information storage section and performs a search in the pattern storage section 17.

Furthermore, in a fourth modification, as shown in Fig. 17, the image display section 16 has a GUI function, and, when the operator specifies either one of the treatment instruments 30A and 30B on the image display section 16, selection information indicating the specified treatment instrument 30A or treatment instrument 30B is input to the swivel control section 14 through the GUI function. In this case, the swivel control section 14 selects the specified treatment instrument 30A or treatment instrument 30B based on the received selection information and drives the rotary microactuators 6 so as to achieve the convergence angle P corresponding to the insertion information of the selected treatment instrument 30A or 30B, included in the control pattern.

By doing so, it is possible to acquire, while automatically giving priority to and automatically switching to, stereoscopic images with a central focus on the treatment region of the treatment part 31 of the desired treatment instrument 30A or treatment instrument 30B that the operator focuses on, among the two treatment instruments 30A and 30B.

In this modification, an information storage section (not shown) that temporarily stores the selection information output from the image display section 16 may be provided. In this case, the swivel control section 14 reads the selection information stored in the information storage section and performs a search in the pattern storage section 17.

### Third Embodiment

Next, a stereo-endoscope robot according to a third embodiment of the present invention will be described.

As shown in Fig. 18, a stereo-endoscope robot 201 of this embodiment includes the stereo-endoscope system 101, two 3D manipulators (manipulation parts) 203A and 203B, and information detecting sections 205A and 205B.

Hereinafter, identical reference symbols are assigned to parts having the same structures as those in the stereo-endoscope device 1 of the first embodiment and the stereo-endoscope system 101 of the second embodiment, and a description thereof will be omitted.

The 3D manipulators 203A and 203B hold the treatment instrument 30A or the treatment instrument 30B to manipulate the treatment instrument 30A or the treatment instrument 30B through remote manipulation performed by the operator. Specifically, with the 3D manipulators 203A and 203B, the operator can perform remote manipulation of the angles of insertion of the trocars 41A and 41B, the amount of insertion of the inserted portion 2, swiveling of the treatment parts 31, and opening and closing of the forceps members 35.

Manipulation information about manipulation of the treatment instruments 30A and 30B performed with the 3D manipulators 203A and 203B is input to the information detecting sections 205A and 205B, respectively.

The manipulation information includes the amounts of insertion of the treatment instruments 30A and 30B, the swivel angles of the swiveling parts 33, and the angles of the trocars 41A and 41B.

The information detecting sections 205A and 205B detect the insertion information of the treatment instruments 30A and 30B, respectively, from the received manipulation information and output it to the swivel control section 14.

The thus-structured endoscope robot 201 of this embodiment can treat the affected site in the subject's body with the 3D manipulators 203A and 203B through remote manipulation performed by the operator. In this case, the swivel control section 14 controls the angles of the swiveling members 5 based on the insertion information of the treatment instrument 30A or 30B, which is detected from the manipulation information of the corresponding one of the 3D manipulators 203A and 203B, thereby making it possible to automatically acquire stereoscopic images with a central focus on the treatment region of the treatment part 31 of the treatment instrument 30A or 30B through remote manipulation.

Although the embodiments of the present invention have been described above in detail with reference to the drawings, the specific structures are not limited to the embodiments, and design changes etc. that do not depart from the gist of the present invention are also encompassed. For example, the present invention is not limited to the above-described embodiments and modifications and may be applied to an embodiment in which these embodiments and modifications are appropriately combined; it is not particularly limited. Furthermore, in the above-described embodiments, the convergence angle α varies linearly with respect to the detected distance; however, it is not limited thereto, and the convergence angle α may vary in a curved manner or vary in a stepwise manner.

Furthermore, in the above-described embodiments, the relationship between the convergence angle P and the amount of insertion of the treatment instrument 30, which is converted to the distance from the treatment part 31 to the imaging devices 7 and associated with the convergence angle, is stored in the pattern storage section 17; however, the distance sensor 9 and the tip distance sensor 10 may include a measurement part for measuring the amount of insertion of the treatment part 31 of the treatment instrument 30, 30A, or 30B inserted into the subject's body and a conversion part for converting the amount of insertion measured by the measurement part to the distance from the treatment part 31 of the treatment instrument 30, 30A, or 30B to the imaging devices 7, and the relationship between the distance and the convergence angle β may be stored in the pattern storage section 17. Even in this case, it is possible to easily detect the distance from the treatment part 31 of the treatment instrument 30, 30A, or 30B to the imaging devices 7 based on the amount of insertion of the treatment part 31, without directly measuring this distance.

Furthermore, in the above-described embodiments, the two swiveling members 5 swivel at the same angle in the opposite directions in synchronization with each other; however, instead of this, they may swivel independently at different angles. By doing so, it is possible not only to perform front-view observation of an object A that is located in front of the inserted portion 2 in the longitudinal direction thereof but also to perform inclined-view observation or sideview observation of an object A that is inclined with respect to the longitudinal direction thereof. Furthermore, only one of the swiveling members 5 may be made to swivel. Furthermore, both of the swiveling members 5 may be made to swivel at different angles or swivel in the same direction.

### {Reference Signs List}

α convergence angle (relative angle)
β convergence angle (relative angle)
1 stereo-endoscope device
2 inserted portion
5 swiveling member (angle changing mechanism)
6 rotary microactuator (driving unit, angle changing mechanism)
7 imaging device
7a optical axis
9 distance sensor (distance detecting unit)
10 tip distance sensor (distance detecting unit)
14 swivel control section (control section)
30, 30A, 30B treatment instrument (medical device)
33 swiveling part
41A, 41B trocar (inserted portion; inserted member)
115 motion detecting section
203A, 203B 3D manipulator
205A, 205B information detecting section
201 stereo-endoscope robot

## Claims

1. A stereo-endoscope system which includes a medical device (30) and a stereo-endoscope device (1), wherein the medical device (30) comprises:
a treatment part (31) that is inserted into a subject's body and that is used to treat an affected site;
a swiveling part (33) that is capable of swiveling the treatment part (31) on a shaft (39) extending in a direction intersecting with the longitudinal axis of the medical device (1); and
a swivel-angle detecting section that detects a swivel angle of the swiveling part (33), and
the stereo-endoscope device (1) comprises:
an inserted portion (2) that can be inserted into the subject's body;
two imaging devices (7) that are disposed on the inserted portion (2) with a space therebetween;
an angle changing mechanism that changes a relative angle of optical axes of the imaging devices (7);
a distance detecting unit (9, 23)
for detecting the distance to the imaging devices (7) from the treatment part (31); and
a control section (14) that controls the angle changing mechanism based on insertion information of the stereo-endoscope system, the insertion information including the distance detected by the distance detecting unit (9) and the swivel angle detected by the swivel-angle detecting section.

2. A stereo-endoscope system according to claim 1, wherein the distance detecting unit (9) includes a measurement part that measures the amount of insertion of the treatment part (31) inserted into the subject's body and a conversion part that converts the amount of insertion measured by the measurement part to the distance from the treatment part (31) to the imaging devices (7).

3. A stereo-endoscope system according to claim 1 or 2, wherein the control section (14) controls the angle changing mechanism such that an intersection of the optical axes of the imaging devices (7) coincides with the position of the treatment part (31).

4. A stereo-endoscope system according to one of claims 1 to 3,
wherein the angle changing mechanism includes, at a tip of the inserted portion (2), a pair of swiveling members (5) attached so as to be capable of swiveling on shafts (4) that extend in a direction intersecting with the longitudinal axis of the inserted portion (2) and driving units (6) for swiveling the pair of swiveling members (5); and
the imaging devices (7) are attached to the swiveling members (5).

5. A stereo-endoscope system according to claim 4, wherein the driving units (6) swivel the pair of swiveling members (5) between closed positions in which the pair of swiveling members (5) extend in the direction of the longitudinal axis of the inserted portion (2) and are close to each other and open positions in which the pair of swiveling members (5) extend in directions intersecting with the longitudinal axis of the inserted portion (2) and are away from each other.

6. A stereo-endoscope system according to one of claims 1 to 5" further comprising:
an inserted member that has a through-hole (21) into which the medical device (30) can be inserted; and
an insertion-angle detecting section that detects an angle of a central axis of the through-hole (21) of the inserted member (2),
wherein the control section (14) controls the angle changing mechanism based on insertion information of the medical device (30), the insertion information including the angle detected by the insertion-angle detecting section.

7. A stereo-endoscope system according to one of claims 1 to 6, further comprising:
at least one of the medical devices (30); and
a motion detecting section that detects motions of the treatment parts (31) of the medical devices (30),
wherein the control section (14) controls the angle changing mechanism based on the distance to the imaging devices (7) from the treatment part (31) of the medical device (30) whose treatment part has the largest amount of motion detected by the motion detecting section.

8. A stereo-endoscope robot comprising:
a stereo-endoscope system (1) according to one of claims 1 to 7;
a manipulation part that manipulates the medical device (30) through remote manipulation performed by an operator; and
an information detecting section that detects the insertion information of the medical device (30) from manipulation information of the medical device (30) manipulated by the manipulation part,
wherein the control section (14) controls the angle changing mechanism based on the insertion information of the medical device (30) detected by the information detecting section.

## Patentansprüche

1. Stereoendoskopsystem, das eine medizinische Vorrichtung (30) und eine Stereoendoskopvorrichtung (1) beinhaltet, wobei die medizinische Vorrichtung (30) aufweist:
ein Behandlungsteil (31), das in einen Körper eines Patienten eingeführt wird und das dazu verwendet wird, eine betroffene Stelle zu behandeln;
ein Schwenkteil (33), das dazu ausgebildet ist, das Behandlungsteil (31) auf einer Welle (39) zu schwenken, die sich in einer Richtung erstreckt, die die Längsachse der medizinischen Vorrichtung (1) schneidet; und
einen Schwenkwinkel-Erkennungsbereich, der einen Schwenkwinkel des Schwenkteils (33) erkennt, und
wobei die Stereoendoskopvorrichtung (1) aufweist:
einen Einführbereich (2), der in den Körper des Patienten eingeführt werden kann;
zwei Bildgebungsvorrichtungen (7), die mit einem Zwischenraum dazwischen an dem Einführbereich (2) angeordnet sind;
einen Winkeländerungsmechanismus, der einen relativen Winkel von optischen Achsen der Bildgebungsvorrichtungen (7) ändert;
eine Abstandserkennungseinheit (9, 23) zum Erkennen des Abstands von dem Behandlungsteil (31) zu den Bildgebungsvorrichtungen (7); und
einen Steuerbereich (14), der den Winkeländerungsmechanismus auf Grundlage von Einführinformationen des Stereoendoskopsystems steuert, wobei die Einführinformationen den Abstand, der durch die Abstandserkennungseinheit (9) erkannt worden ist, und den Schwenkwinkel beinhalten, der durch den Schwenkwinkel-Erkennungsbereich erkannt worden ist.

2. Stereoendoskopsystem nach Anspruch 1, wobei die Abstandserkennungseinheit (9) ein Messteil, das den Einführbetrag des Behandlungsteils (31) misst, das in den Körper des Patienten eingeführt worden ist, und ein Umrechnungsteil beinhaltet, das den durch das Messteil gemessenen Einführbetrag in den Abstand von dem Behandlungsteil (31) zu den Bildgebungsvorrichtungen (7) umrechnet.

3. Stereoendoskopsystem nach Anspruch 1 oder 2, wobei der Steuerbereich (14) den Winkeländerungsmechanismus so steuert, dass ein Schnittpunkt der optischen Achsen der Bildgebungsvorrichtungen (7) mit der Position des Behandlungsteils (31) übereinstimmt.

4. Stereoendoskopsystem nach einem der Ansprüche 1 bis 3,
wobei der Winkeländerungsmechanismus an einer Spitze des Einführbereichs (2) ein Paar Schwenkelemente (5), das so angebracht ist, dass es in der Lage ist, auf Wellen (4) zu schwenken, die sich einer Richtung erstrecken, die die Längsachse des Einführbereichs (2) schneidet, und Antriebseinheiten (6) zum Schwenken des Paares Schwenkelemente (5) beinhaltet; und
die Bildgebungsvorrichtungen (7) an den Schwenkelementen (5) angebracht sind.

5. Stereoendoskopsystem nach Anspruch 4, wobei die Antriebseinheiten (6) das Paar Schwenkelemente (5) zwischen geschlossenen Stellungen, in denen das Paar Schwenkelemente (5) sich in der Richtung der Längsachse des Einführbereichs (2) erstreckt und einander nahe gelegen ist, und geöffneten Stellungen schwenken, in denen das Paar Schwenkelemente (5) sich in Richtungen erstreckt, die die Längsachse des Einführbereichs (2) schneiden und die voneinander entfernt sind.

6. Stereoendoskopsystem nach einem der Ansprüche 1 bis 5, das ferner aufweist:
ein Einführelement, das eine Durchgangsöffnung (21) aufweist, in die die medizinische Vorrichtung (30) eingeführt werden kann; und
einen Einführwinkel-Erkennungsbereich, der einen Winkel einer Mittelachse der Durchgangsöffnung (21) des Einführelements (2) erkennt,
wobei der Steuerbereich (14) den Winkeländerungsmechanismus auf Grundlage von Einführinformationen der medizinischen Vorrichtung (30) steuert, wobei die Einführinformationen den Winkel beinhalten, der durch den Einführwinkel-Erkennungsbereich erkannt worden ist.

7. Stereoendoskopsystem nach einem der Ansprüche 1 bis 6, das ferner aufweist:
mindestens eine der medizinischen Vorrichtungen (30); und
einen Bewegungserkennungsbereich, der Bewegungen der Behandlungsteile (31) der medizinischen Vorrichtungen (30) erkennt,
wobei der Steuerbereich (14) den Winkeländerungsmechanismus auf Grundlage des Abstands von dem Behandlungsteil (31) der medizinischen Vorrichtung (30), deren Behandlungsteil den größten durch den Bewegungserkennungsbereich erkannten Bewegungsbetrag aufweist, zu den Bildgebungsvorrichtungen (7) steuert.

8. Stereoendoskoproboter, der aufweist:
ein Stereoendoskopsystem (1) nach einem der Ansprüche 1 bis 7;
ein Manipulationsteil, das die medizinische Vorrichtung (30) durch eine durch einen Bediener durchgeführte Manipulation aus der Ferne manipuliert; und
einen Informationserkennungsbereich, der die Einführinformationen der medizinischen Vorrichtung (30) aus Manipulationsinformationen der medizinischen Vorrichtung (30) erkennt, die durch das Manipulationsteil manipuliert wird,
wobei der Steuerbereich (14) den Winkeländerungsmechanismus auf Grundlage der Einführinformationen der medizinischen Vorrichtung (30) steuert, die durch den Informationserkennungsbereich erkannt worden sind.

## Revendications

1. Système d'endoscope stéréoscopique qui comprend un dispositif médical (30) et un dispositif d'endoscope stéréoscopique (1), dans lequel le dispositif médical (30) comprend :
une partie de traitement (31) qui est insérée dans le corps d'un sujet et qui est utilisée pour traiter un site affecté ;
une partie pivotante (33) qui est capable de faire pivoter la partie de traitement (31) sur un arbre (39) s'étendant dans une direction croisant l'axe longitudinal du dispositif médical (30) ; et
une section de détection d'angle de pivotement qui détecte un angle de pivotement de la partie pivotante (33), et
le dispositif d'endoscope stéréoscopique (1) comprend :
une partie insérée (2) qui peut être insérée dans le corps du sujet ;
deux dispositifs d'imagerie (7) qui sont disposés sur la partie insérée (2) avec un espace entre eux ;
un mécanisme de changement d'angle qui change un angle relatif des axes optiques des dispositifs d'imagerie (7) ;
une unité de détection de distance (9, 23) destinée à détecter la distance entre la partie de traitement (31) et les dispositifs d'imagerie (7) ; et
une section de commande (14) qui commande le mécanisme de changement d'angle sur la base des informations d'insertion du système d'endoscope stéréoscopique, les informations d'insertion incluant la distance détectée par l'unité de détection de distance (9) et l'angle de pivotement détecté par la section de détection d'angle de pivotement.

2. Système d'endoscope stéréoscopique selon la revendication 1, dans lequel l'unité de détection de distance (9) comprend une partie de mesure qui mesure la quantité d'insertion de la partie de traitement (31) insérée dans le corps du sujet et une partie de conversion qui convertit la quantité d'insertion mesurée par la partie de mesure en distance allant de la partie de traitement (31) aux dispositifs d'imagerie (7).

3. Système d'endoscope stéréoscopique selon la revendication 1 ou 2, dans lequel la section de commande (14) commande le mécanisme de changement d'angle d'une manière telle qu'une intersection des axes optiques des dispositifs d'imagerie (7) coïncide avec la position de la partie de traitement (31).

4. Système d'endoscope stéréoscopique selon l'une quelconque des revendications 1 à 3,
dans lequel le mécanisme de changement d'angle comprend, au niveau d'une pointe de la partie insérée (2), une paire d'éléments pivotants (5) fixés de façon à être capables de pivoter sur des arbres (4) qui s'étendent dans une direction croisant l'axe longitudinal de la partie insérée (2) et des unités d'entraînement (6) pour faire pivoter la paire d'éléments pivotants (5) ; et
les dispositifs d'imagerie (7) sont fixés aux éléments pivotants (5).

5. Système d'endoscope stéréoscopique selon la revendication 4, dans lequel les unités d'entraînement (6) font pivoter les éléments pivotants (5) formant une paire entre des positions fermées dans lesquelles les éléments pivotants (5) formant une paire s'étendent dans la direction de l'axe longitudinal de la partie insérée (2) et sont proches l'un de l'autre et des positions ouvertes dans lesquelles les éléments pivotants (5) formant une paire s'étendent dans des directions croisant l'axe longitudinal de la partie insérée (2) et sont espacés l'un de l'autre.

6. Système d'endoscope stéréoscopique selon l'une quelconque des revendications 1 à 5, comprenant en outre :
un élément inséré qui comporte un trou traversant (21) dans lequel le dispositif médical (30) peut être inséré ; et
une section de détection d'angle d'insertion qui détecte un angle d'un axe central du trou traversant (21) de l'élément inséré (2),
dans lequel la section de commande (14) commande le mécanisme de changement d'angle sur la base des informations d'insertion du dispositif médical (30), les informations d'insertion incluant l'angle détecté par la section de détection d'angle d'insertion.

7. Système d'endoscope stéréoscopique selon l'une quelconque des revendications 1 à 6, comprenant en outre :
au moins l'un parmi les dispositifs médicaux (30) ; et
une section de détection de mouvement qui détecte les mouvements des parties de traitement (31) des dispositifs médicaux (30),
dans lequel la section de commande (14) commande le mécanisme de changement d'angle sur la base de la distance entre les dispositifs d'imagerie (7) et la partie de traitement (31) du dispositif médical (30) dont la partie de traitement présente la quantité de mouvement la plus importante détectée par la section de détection de mouvement.

8. Robot endoscopique stéréoscopique comprenant :
un système d'endoscope stéréoscopique (1) selon l'une quelconque des revendications 1 à 7 ;
une partie de manipulation qui manipule le dispositif médical (30) par l'intermédiaire d'une manipulation à distance exécutée par un opérateur ; et
une section de détection d'informations qui détecte les informations d'insertion du dispositif médical (30) à partir des informations de manipulation du dispositif médical (30) manipulé par la partie de manipulation,
dans lequel la section de commande (14) commande le mécanisme de changement d'angle sur la base des informations d'insertion du dispositif médical (30) détectées par la section de détection d'informations.
